(19)

Europäisches
Patentamt
European
Patent Office
Office européen
des brevets

(11) **EP 3 918 996 A1**

(12) **EUROPEAN PATENT APPLICATION**

published in accordance with Art. 153(4) EPC

(43) Date of publication:
08.12.2021 Bulletin 2021/49

(21) Application number: 20774467.3

(22) Date of filing: 13.03.2020

(51) Int Cl.:
*A61B 5/16* [(2006.01)]  *A61B 5/024* [(2006.01)]

(86) International application number:
PCT/CN2020/079153

(87) International publication number:
WO 2020/187144 (24.09.2020 Gazette 2020/39)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 20.03.2019 CN 201910216114

(71) Applicant: Huawei Technologies Co., Ltd.
Shenzhen, Guangdong 518129 (CN)

(72) Inventors:
• FU, Xiaoyu
Shenzhen, Guangdong 518129 (CN)
• XU, Peida
Shenzhen, Guangdong 518129 (CN)
• LI, Yan
Shenzhen, Guangdong 518129 (CN)

(74) Representative: Pfenning, Meinig & Partner mbB
Patent- und Rechtsanwälte
Theresienhöhe 11a
80339 München (DE)

(54) **PSYCHOLOGICAL STRESS ASSESSMENT METHOD AND RELATED DEVICE**

(57) A psychological stress assessment method and a related device are provided. The psychological stress assessment method includes: determining a first psychological stress difference of a target user (S401); determining a second psychological stress difference of a target crowd (S403); and determining a psychological stress status of the target user based on the first psychological stress difference and the second psychological stress difference (S404). First, the psychological stress difference of the target user in two activities of different types is pertinently determined, then the psychological stress difference of the crowd to which the target user belongs in the two activities is determined, and finally the psychological status of the target user is accurately determined. This reduces inaccuracy of an individual difference, and also provides a more effective psychological stress assessment method to improve psychological stress assessment accuracy.

S401

Determine a first psychological stress difference of a target user

S402

Determine, based on user information of the target user, a target crowd to which the target user belongs

S403

Determine a second psychological stress difference of the target crowd

S404

Determine a psychological stress status of the target user based on the first psychological stress difference and the second psychological stress difference

FIG. 4A

EP 3 918 996 A1

## Description

This application claims priority to Chinese Patent Application No. 201910216114.2, filed with the China National Intellectual Property Administration on March 20, 2019 and entitled "PSYCHOLOGICAL STRESS ASSESSMENT METHOD AND RELATED DEVICE", which is incorporated herein by reference in its entirety.

## TECHNICAL FIELD

This application relates to the field of psychological health application technologies, and in particular, to a psychological stress assessment method and a related device.

## BACKGROUND

With continuous development of society and economy, people are increasingly facing stresses from various aspects. If people stay in such a stress environment for a long time, human psychological health is seriously affected, and even human lives are threatened. Therefore, to ensure their psychological health, people know their psychological statuses in time, and usually perform psychological stress assessment by using a psychology-related test method of a statistical model (for example, a test method and scale method in which heart rate variability is combined with a fixed statistical model), or seek help from a psychologist for psychological health assessment, or the like.

However, in the prior art, because the heart rate variability is strongly correlated with an individual, in the method of performing psychological stress assessment by using the heart rate variability in combination with the fixed statistical model, although a psychological stress value can be obtained based on a related physiological feature value, the fixed statistical model used in the method is difficult to well match an actual status of an individual due to a limitation of the model. However, the method of seeking help from the psychologist for assessment is often affected by subjective factors of the psychologist and a testee, and therefore a psychological stress assessment result often does not match a feeling of the testee. Therefore, how to provide a more effective psychological stress assessment method to improve psychological stress assessment accuracy is an urgent problem to be resolved.

## SUMMARY

Embodiments of this application provide a psychological stress assessment method and a related device, to improve psychological stress assessment accuracy.

According to a first aspect, an embodiment of this application provides a psychological stress assessment method. The method may include: determining a first psychological stress difference of a target user, where the first psychological stress difference is an absolute value of a difference between a first psychological stress and a second psychological stress, the first psychological stress is a psychological stress value of the target user in a first activity, the second psychological stress is a psychological stress value of the target user in a second activity, and the first activity and the second activity are activities of different types; determining a second psychological stress difference of a target crowd, where the target crowd is a crowd to which the target user belongs, the second psychological stress difference is an absolute value of a difference between a third psychological stress and a fourth psychological stress, the third psychological stress is an average psychological stress value of the target crowd in the first activity, and the fourth psychological stress is an average psychological stress value of the target crowd in the second activity; and determining a psychological stress status of the target user based on the first psychological stress difference and the second psychological stress difference.

In this embodiment of this application, in the psychological stress assessment method, first, the first psychological stress difference of the target user in the first activity and the second activity is determined, then the second psychological stress difference of the target crowd in the same two activities is determined, and finally the psychological stress status of the target user is determined based on the first psychological stress difference and the second psychological stress difference. In this embodiment of this application, considering an individual difference of a user and a similarity of a user crowd, that is, on a basis that the user feels different psychological stresses in different activities, but members in the crowd to which the user belongs often feel similar psychological stresses in a same activity, first, the psychological stress difference of the target user in the two activities of different types is pertinently determined, then the psychological stress difference of the crowd to which the target user belongs in the two activities is determined, and finally the psychological status of the target user is accurately determined. This reduces inaccuracy of an individual difference, and also provides a more effective psychological stress assessment method to improve psychological stress assessment accuracy.

In a possible implementation, the first psychological stress is a psychological stress value determined based on a heartbeat interval sequence $T_1$ in a first time period, and the first time period is a time period in which the target user performs the first activity; and the second psychological stress is a psychological stress value determined based on a heartbeat interval sequence $T_2$ in a second time period, and the second time period is a time period in which the target user performs the second activity. This embodiment of this application provides a method for determining, by using heartbeat interval sequences of a user in different activities, psychological stresses corresponding to the activities. For example, a plurality of heartbeat interval sequences of the user in a time period in which an activity is performed are first obtained, and then a psychological status of the user is further accurately assessed based on a fluctuation status of the plurality of heartbeat interval sequences, thereby improving psychological stress assessment accuracy.

In a possible implementation, a calculation formula of the first psychological stress is as follows: $Y=\Delta d+\alpha*f(X)$, where Y is the first psychological stress, $\Delta d$ is a preset stress calibration value, $\alpha$ is a target stress gain factor, the target stress gain factor is used to represent a psychological stress change degree of the target user relative to the target crowd, f(X) is a function related to a physiological parameter X, X is determined based on M heartbeat interval sequences $T_1$ in the first time period, X represents heartbeat interval sequence variability of the target user in the first time period, and M is a positive integer. Optionally, a calculation formula of the second psychological stress is the same as the foregoing calculation formula, and a physiological parameter X corresponding to the second psychological stress is determined based on M heartbeat interval sequences $T_2$ in the foregoing second time period. This embodiment of this application provides an assessment method for determining, by using heartbeat interval sequences of a user in different activities, psychological stresses corresponding to the activities. Specifically, first, a physiological parameter X used to represent heartbeat interval sequence variability is first determined based on a plurality of heartbeat interval sequences, then a psychological stress corresponding to the physiological parameter is determined based on a mapping relationship between the physiological parameter X and the psychological stress, and then the psychological stress value is corrected based on a target stress gain factor that is of the user and that is used to represent a psychological stress change degree, so that a psychological status of the user can be accurately assessed, thereby improving accuracy of psychological stress assessment for the user.

In a possible implementation, the target stress gain factor is determined based on $C_G^2$ stress gain factors, $C_G^2$ represents a combination quantity of any two of G activities of different types, the stress gain factor is a ratio of a third psychological stress difference to a fourth psychological stress difference, the third psychological stress difference is an absolute value of a difference between a fifth psychological stress and a sixth psychological stress, the fifth psychological stress and the sixth psychological stress are psychological stress values respectively corresponding to the target user in any two of the G activities, the fourth psychological stress difference is an absolute value of a difference between a seventh psychological stress and an eighth psychological stress, the seventh psychological stress and the eighth psychological stress are average psychological stress values respectively corresponding to the target crowd in the any two activities, and the G activities of different types include the first activity and the second activity, and G is a positive integer greater than or equal to 2. This embodiment of this application provides an assessment method for determining a psychological stress of a target user based on a target stress gain factor. In this method, considering an individual difference of a user, that is, the user feels different psychological stresses in different activities, first, a stress gain factor of any two of a plurality of activities of different types may be determined, a target stress gain factor applicable to any activity type may be determined based on the foregoing plurality of stress gain factors by using a weighted averaging method, and finally the psychological stress of the target user may be determined based on the target stress gain factor. In addition, with an increase in a quantity of types of activities that the user participates in and an increase in duration of an activity that the user participates in, a degree of correlation between the target stress gain factor and the user also increases, so that accuracy of the psychological stress value finally determined by using the target stress gain factor increases, and psychological stress assessment for the user is more accurate.

In a possible implementation, before the determining a second psychological stress difference of a target crowd, the method further includes: determining, based on user information of the target user, the target crowd to which the target user belongs, where the user information includes one or more of an occupation, an age, a sex, a height, a weight, or average daily sleep duration. Optionally, the target user may belong to a plurality of target crowds. It may be understood that in this embodiment of this application, crowd classification may be performed based on the user information, and classification of the target crowd is more accurate when the user information is more detailed. This embodiment of this application provides an assessment method for determining a psychological stress of a target user based on a target crowd. Due to a similarity of a user crowd, a psychological stress of the target user is more similar to that of a user of a same type than a user of a different type. Therefore, when the target crowd has a higher similarity degree, the target crowd also has greater reference value for the user. Therefore, in this embodiment of this application, crowd classification is performed according to a specific rule by using the user information of the user, so that the crowd to which the user belongs has a higher similarity degree when the user information is more detailed. Therefore, after the target crowd is determined by using the classification manner, an obtained average psychological stress of the target crowd is more accurate, and further a psychological stress that is of the user and that is determined based on the average psychological stress is also more accurate.

In a possible implementation, the target crowd includes N users; the third psychological stress is an average psychological stress determined based on psychological stress values of the N users in a third time period, the third time period is a time period in which the N users perform the first activity, and N is a positive integer; and the fourth psychological stress is an average psychological stress value determined based on psychological stress values of the N users in a fourth time period, and the fourth time period is a time period in which the N users perform the second activity. This embodiment of this application provides an assessment method for determining a psychological stress of a target user based on a target crowd. In this method, considering an individual difference of a user, users with a very high similarity degree in the crowd may have different psychological stresses in activities of a same activity type. Therefore, an average psychological stress of users in the crowd is determined based on psychological stress values of the plurality of users in the crowd, and further the psychological stress of the target user is determined by using the average psychological stress, so that psychological stress assessment for the user is more accurate.

According to a second aspect, an embodiment of this application provides a psychological stress assessment apparatus, including:

a first determining unit, configured to determine a first psychological stress difference of a target user, where the first psychological stress difference is an absolute value of a difference between a first psychological stress and a second psychological stress, the first psychological stress is a psychological stress value of the target user in a first activity, the second psychological stress is a psychological stress value of the target user in a second activity, and the first activity and the second activity are activities of different types;

a second determining unit, configured to determine a second psychological stress difference of a target crowd, where the target crowd is a crowd to which the target user belongs, the second psychological stress difference is an absolute value of a difference between a third psychological stress and a fourth psychological stress, the third psychological stress is an average psychological stress value of the target crowd in the first activity, and the fourth psychological stress is an average psychological stress value of the target crowd in the second activity; and

a third determining unit, configured to determine a psychological stress status of the target user based on the first psychological stress difference and the second psychological stress difference.

In this embodiment of this application, in the psychological stress assessment apparatus, first, the first determining unit determines the first psychological stress difference of the target user in the first activity and the second activity, then the second determining unit determines the second psychological stress difference of the target crowd in the same two activities, and finally the third determining unit determines the psychological stress status of the target user based on the first psychological stress difference and the second psychological stress difference. Due to an individual difference of a user, the user may feel non-uniform psychological stresses in different activities. Therefore, in this embodiment of this application, the psychological stress assessment apparatus first pertinently determines the psychological stress difference of the target user in the two activities of different types, then determines the psychological stress difference of the crowd to which the target user belongs in the two activities, and finally accurately determines the psychological status of the target user. This reduces inaccuracy of an individual difference, and improves psychological stress assessment accuracy.

In a possible implementation, the first psychological stress is a psychological stress value determined based on a heartbeat interval sequence $T_1$ in a first time period, and the first time period is a time period in which the target user performs the first activity; and the second psychological stress is a psychological stress value determined based on a heartbeat interval sequence $T_2$ in a second time period, and the second time period is a time period in which the target user performs the second activity. This embodiment of this application provides a psychological assessment apparatus that obtains a heartbeat interval sequence of a target user in a time period in which an activity is performed, and then further determines a psychological stress value of the target user based on the heartbeat interval sequence. The apparatus can accurately assess a psychological status of the target user, to reduce an impact on a final assessment result due to a subjective factor of the target user, thereby improving psychological stress assessment accuracy.

In a possible implementation, a calculation formula of the first psychological stress is as follows: $Y=\Delta d+\alpha*f(X)$, where Y is the first psychological stress, $\Delta d$ is a preset stress calibration value, $\alpha$ is a target stress gain factor, the target stress gain factor is used to represent a psychological stress change degree of the target user relative to the target crowd, f(X) is a function related to a physiological parameter X, X is determined based on M heartbeat interval sequences $T_1$ in the first time period, X represents heartbeat interval sequence variability of the target user in the first time period, and M is a positive integer.

In a possible implementation, the target stress gain factor is determined based on $C_G^2$ stress gain factors, $C_G^2$ represents a combination quantity of any two of G activities of different types, the stress gain factor is a ratio of a third psychological stress difference to a fourth psychological stress difference, the third psychological stress difference is an absolute value of a difference between a fifth psychological stress and a sixth psychological stress, the fifth psychological stress and the sixth psychological stress are psychological stress values respectively corresponding to the target user in any two of the G activities, the fourth psychological stress difference is an absolute value of a difference between a seventh psychological stress and an eighth psychological stress, the seventh psychological stress and the eighth psychological stress are average psychological stress values respectively corresponding to the target crowd in the any two activities, and the G activities of different types include the first activity and the second activity, and G is a positive integer greater than or equal to 2.

In a possible implementation, the apparatus further includes a fourth determining unit, configured to: before the second psychological stress difference of the target crowd is determined, determine, based on user information of the target user, the target crowd to which the target user belongs, where the user information includes one or more of an occupation, an age, a sex, a height, a weight, or average daily sleep duration. In a possible implementation, the target crowd includes N users; the third psychological stress is an average psychological stress determined based on psychological stress values of the N users in a third time period, the third time period is a time period in which the N users perform the first activity, and N is a positive integer; and the fourth psychological stress is an average psychological stress value determined based on psychological stress values of the N users in a fourth time period, and the fourth time period is a time period in which the N users perform the second activity.

According to a third aspect, an embodiment of this application provides a terminal device. The terminal device includes a processor. The processor is configured to support the terminal device in implementing the corresponding function in the psychological stress assessment method provided in the first aspect. The terminal device may further include a memory. The memory is coupled to the processor, and stores a program instruction and data that are necessary for the terminal device. The terminal device may further include a communications interface, used by the terminal device to communicate with another device or a communications network.

According to a fourth aspect, an embodiment of this application provides a computer storage medium, configured to store a computer software instruction used by a processor in the psychological stress assessment apparatus provided in the second aspect. The computer software instruction includes a program designed for executing the foregoing aspect.

According to a fifth aspect, an embodiment of this application provides a computer program. The computer program includes instructions. When the computer program is executed by a computer, the computer is enabled to execute a procedure executed by a processor in the psychological stress assessment apparatus in the second aspect.

According to a sixth aspect, this application provides a chip system. The chip system includes a processor, configured to support a network device in implementing the function in the first aspect, for example, generating or processing the information in the psychological stress assessment method. In a possible design, the chip system further includes a memory. The memory is configured to store a program instruction and data that are necessary for the data sending device. The chip system may include a chip, or may include a chip and another discrete device.

## BRIEF DESCRIPTION OF DRAWINGS

To describe the technical solutions in the embodiments of this application or in the background more clearly, the following describes the accompanying drawings required for describing the embodiments of this application or the background.

FIG. 1 is a schematic change diagram of psychological stresses of a user A and a user B according to an embodiment of this application;

FIG. 2 is a schematic architectural diagram of a psychological stress assessment system according to an embodiment of this application;

FIG. 3 is a schematic interface diagram of an example client 202 according to an embodiment of this application;

FIG. 4A is a schematic flowchart of a psychological stress assessment method according to an embodiment of this application;

FIG. 4B is a schematic diagram of an application scenario of a user A during walking according to an embodiment of this application;

FIG. 4C is a schematic diagram of an application scenario of a user A during sleeping according to an embodiment of this application;

FIG. 4D is a schematic diagram of an application scenario of a user A during bungee jumping according to an embodiment of this application;

FIG. 4E is a schematic diagram of an interface for determining a target crowd according to an embodiment of this application;

FIG. 5 is a schematic diagram of a psychological stress assessment apparatus according to an embodiment of this application; and

FIG. 6 is a schematic structural diagram of another psychological stress assessment apparatus according to an embodiment of this application.

## DESCRIPTION OF EMBODIMENTS

The following describes the embodiments of this application with reference to the accompanying drawings in the embodiments of this application.

In the specification, claims, and accompanying drawings of this application, the terms such as "first", "second", "third", "fourth", "fifth", "sixth", "seventh", and "eighth" are intended to distinguish between different objects but do not describe a particular order. In addition, the terms "include", "have", and any other variant thereof, are intended to cover a non-exclusive inclusion. For example, a process, a method, a system, a product, or a device that includes a series of steps or units is not limited to the listed steps or units, but optionally further includes an unlisted step or unit, or optionally further includes another step or unit inherent to the process, the method, the product, or the device.

Mentioning an "embodiment" in the specification means that a particular characteristic, structure, or feature described with reference to the embodiment may be included in at least one embodiment of this application. The phrase shown in various locations in the specification may be not necessarily a same embodiment, or an independent or optional embodiment exclusive from another embodiment. It is explicitly and implicitly understood by persons skilled in the art that the embodiment described in the specification may be combined with another embodiment.

Terms such as "component", "module", and "system" used in this specification are used to represent computer-related entities, hardware, firmware, combinations of hardware and software, software, or software being executed. For example, a component may be but is not limited to a process that runs on a processor, a processor, an object, an executable file, a thread of execution, a program, and/or a computer. As shown in figures, both an application that runs on a computing device and the computing device may be components. One or more components may reside in a process and/or a thread of execution, and the components may be located in one computer and/or distributed between two or more computers. In addition, these components may be executed from various computer-readable media that store various data structures. For example, the components may perform communication by using a local and/or remote process and based on a signal having one or more data packets (for example, data from two components interacting with another component in a local system, a distributed system, and/or across a network such as the Internet interacting with another system by using the signal).

It should be understood that the term "and/or" used in the specification and the appended claims of this application indicates any combination and all possible combinations of one or more of associated listed items, and includes these combinations.

First, some terms in this application are described, to help persons skilled in the art have a better understanding.

(1) Pulse wave: The pulse wave is generated through propagation of heart pulsation (vibration) to the periphery along arterial blood vessels and blood flows. Therefore, a propagation speed of the pulse wave depends on physical and geometric properties of propagation media, including arterial elasticity, a lumen size, blood density and viscosity, and the like, and is especially closely related to arterial wall elasticity, an arterial wall diameter, and an arterial wall thickness. It is found, through experiments, that greater arterial blood vessel elasticity (namely, greater arterial blood vessel compliance) indicates a lower propagation speed of the pulse wave, and a smaller arterial diameter indicates a higher speed. Therefore, the pulse wave propagates more quickly from a main artery to a large artery and then to a smaller artery.

(2) Electrocardiogram (ElectroCardioGram, ECG for short): The ECG is an objective indicator of occurrence, propagation, and recovery processes of heart excitation.

(3) Cardiac cycle (Cardiac Cycle): The cardiac cycle is a process experienced by a cardiovascular system from a start of a heartbeat to a start of a next heartbeat. Both a systole period and a diastole period are shortened as a heart rate increases, but the diastole period is shorted at a larger proportion. Ventricular systole is a main blood flow pushing force. Conventionally, a start and a stop of the ventricular systole are used as signs of the cardiac cycle, a ventricular systole period is referred to as the systole period, and a ventricular diastole period is referred to as the diastole period. Duration of one cardiac cycle is determined based on a heart rate. If the heart rate is 75 times per minute, duration for completing one cardiac cycle is 0.8s.

(4) Heart rate (Heart Rate): The heart rate is a professional term used to describe a cardiac cycle, indicates a quantity of heartbeat times per minute, and is subject to a first sound. The heart rate is interpreted in modern Chinese as "heartbeat frequency". The frequency is a quantity of times that an event occurs in a unit time. The two explanations are combined to mean a quantity of heartbeat times within a specific time, namely, a heartbeat speed within the specific time.

(5) Definition and calculation formula of a combination: M ($m \leq n$) elements are randomly selected from n different elements to constitute a group, referred to as a combination of the m elements selected from the n different elements. A quantity of all combinations of the m ($m \leq n$) elements selected from the n different elements is referred to as a combination quantity of the m elements selected from the n different elements.

(6) Psychological stress: The psychological stress is a physiological and psychological tension state in a life adaptation process of an individual, and stems from an imbalance between an environment requirement and a coping capability of the individual. This tension state tends to be manifested through non-specific psychological and physiological responses. A stress is a type of cognitive and behavioral experience including both a stressor and a stress response. A human inner conflict and accompanying emotional experience are a psychological stress, also referred to as a mental stress.

(7) Heart rate variability (Heart Rate Variability, HRV): The HRV indicates a small difference in an interval between successive heartbeats, and is generated by modulating a heart sinus node by an autonomic nervous system, so that a difference and fluctuation of dozens of milliseconds usually exist in the heartbeat interval. The heart rate variability indicates a change status of a period difference between successive heartbeats, and includes information about regulating a cardiovascular system by a neurohumoral factor. The heart rate variability is used to reflect activeness of the autonomic nervous system and quantitatively assess tension and balance of a cardiac sympathetic nerve and a vagus nerve, to determine a condition and prevention of a cardiovascular disease. The heart rate variability may be a valuable index for predicting sudden cardiac death and arrhythmia events.

(8) Aerobic exercise: The aerobic exercise indicates any rhythmic exercise, and has a relatively long exercise time (about 15 minutes or more) and medium or upper exercise intensity (a maximum heart rate value of 60% to 80%). The aerobic exercise is a constant exercise that lasts for at least five minutes while energy is still remained.

(9) Anaerobic exercise: The anaerobic exercise indicates a high-speed and vigorous exercise in a "hypoxia" state of muscles. Most of anaerobic exercises are exercises with high load intensity and strong instantaneousness, and therefore are difficult to last for a long time and also need a long fatigue elimination time. When a done exercise is very vigorous or bursts out rapidly, such as weightlifting, 100-meter sprint, or wrestling, a body needs a lot of energy in an instant. However, under a normal circumstance, aerobic metabolism cannot meet a need of the body at this time, and then anaerobic metabolism is performed based on sugar to quickly produce a lot of energy. An exercise in this state is the anaerobic exercise.

Next, to facilitate understanding of the embodiments of the present invention, the following specifically analyzes a technical problem that needs to be resolved in the embodiments of the present invention and a corresponding application scenario. The following two test solutions are used in most of psychological stress assessment processes.

Solution 1: performing psychological stress assessment by using a psychology-related test method of a statistical model (for example, a test method and scale method in which heart rate variability is combined with a fixed statistical model). Solution 2: Seeking help from a psychologist for psychological health assessment, or the like.

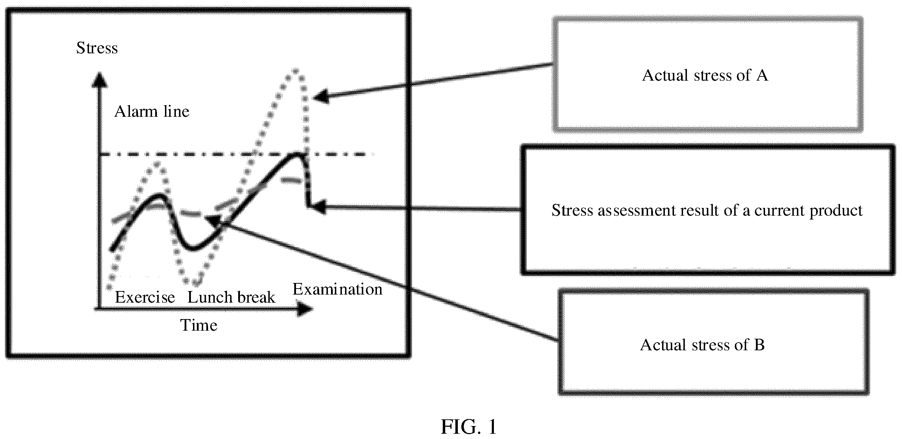

In the solution 1, psychological stress assessment is performed by using the heart rate variability HRV in combination with the fixed statistical model. For example, in a following application scenario, a 25-year-old IT practitioner A who never exercises and severely lacks sleep and a 40-year-old retired officer B who regularly exercises and sleeps well experience a same exercise-lunch break-examination activity process. FIG. 1 is a schematic change diagram of psychological stresses of the user A and the user B according to an embodiment of this application. It can be clearly seen from FIG. 1 that when psychological stress assessment is performed by using the current heart rate variability HRV in combination with the fixed statistical model, a stress assessment result curve of the IT practitioner A may be the same as that of the retired officer B. However, actually, there is a high probability that an anti-stress capability of B is stronger than that of A, and therefore B actually feels less stress fluctuation than A. This case occurs for the following reason: In the foregoing example, when psychological stresses of the IT practitioner A and the retired officer B are calculated, pulse waves PPGs or electrocardiograms ECGs of the IT practitioner A and the retired officer B are measured, that is, R waves in the ECGs are obtained; and further, heartbeat RR (instantaneous heart rate) time intervals are determined based on the R waves and the heartbeat RR time intervals are determined as sequences of numbers, then physiological feature values corresponding to the IT practitioner A and the retired officer B are obtained, and finally a related stress calculation model is obtained in a machine learning manner, to obtain, through calculation, the psychological stress values corresponding to the IT practitioner A and the retired officer B. In this method, although a psychological stress value can be obtained based on a related physiological feature value, the fixed statistical model used in the method is difficult to well match an actual status of an individual due to a limitation of the model. Further, due to particularity of a psychological problem, heartbeat RR also has a significant individual difference, that is, psychological stress values Y corresponding to same heartbeat RR are also strongly correlated with individuals, and cannot be described by using a single model.

However, currently, most of commonly used statistical models are fixed models, and therefore have insufficient personalization degrees, causing a deviation between a stress assessment result and a subjective feeling. Therefore, a stress assessment result often does not match a feeling of a user, and consequently a psychological stress status of the user cannot be accurately determined.

In the solution 2, help is sought from the psychologist for psychological health assessment. Due to an individual difference of a user, different users may have different psychological stresses for a same symptom, and each psychologist has a different professional direction, different work experience, and the like. Therefore, different psychologists may have different determining results for a same user with a same symptom. Therefore, a psychological stress assessment result also often does not match a feeling of a user. Further, because a psychologist cannot observe a psychological status of a user anytime or anywhere, the psychologist cannot assess the psychological status of the user in time.

Therefore, for the foregoing technical problem, a problem mainly resolved in this application is how to provide, on a basis that a psychological stress is tested by using a fixed statistical model, a more accurate and objective psychological stress calculation manner for users with different attribute features in different states, so that a psychological stress of a user is more accurately and effectively calculated.

The following lists examples of a scenario to which the psychological stress assessment method in this application is applied, and the scenario may include the following two scenarios:

Scenario 1: helping a user know a psychological stress status of the user in time, to maintain psychological health of the user.

Modern medicine has proven that a psychological stress may weaken an immune system of a human body, and consequently a body disease is caused by an external pathogenic factor. A stress beyond psychological tolerance may cause a psychological disease such as psychological imbalance, depression, or anxiety, or may cause a person to have the following symptoms: tachycardia, cold or sweaty palms, shortness of breath, headache/stomachache, nausea/emesis, abdominal distention/diarrhea, muscle tingling, amnesia/insomnia, low self-esteem, suspicion, jealousy, despondency, confused thinking, grumpiness, overexcitement, moodiness, and the like. If a psychological stress can be accurately obtained and can be monitored in real time without perception, and a reminder and an alarm can be provided in time when the stress is high, or further a related psychological health service can be customized based on a requirement of the user, so that the user can adjust the psychological stress in time before the psychological stress causes a body damage. This is of great significance for keeping body health. Therefore, the user needs to know the psychological stress status of the user in time, to maintain the psychological health of the user.

Scenario 2: understanding livelihood and conditions of the people based on psychological health of the people, to help the country develop.

Assuming that a psychological health degree of a user is determined based on a psychological stress status in a specific period, a related team can know whether a psychological stress status of a member in the team is at a normal level in a crowd to which the member belongs, and then pay attention to working and life of the member in time, to guide the member to adjust psychological health of the member in time. Alternatively, a related team such as an enterprise or a school, or a related researcher may be helped understand psychological health requirements of different regions and different users in work and study, so that the related team or the related researcher can find a psychological problem of a member in time, to avoid a tragedy caused by an excessive psychological stress. Further, the livelihood and conditions of the people even can be known based on the psychological health of the people, to help the country develop.

It may be understood that the foregoing two application scenarios are merely example implementations in the embodiments of this application, and the application scenario in the embodiments of this application includes but is not limited to the foregoing application scenarios.

The following specifically analyzes and resolves the technical problem in the foregoing solutions with reference to a psychological stress assessment system architecture provided in this application and based on a psychological stress assessment method process provided based on the psychological stress assessment system architecture.

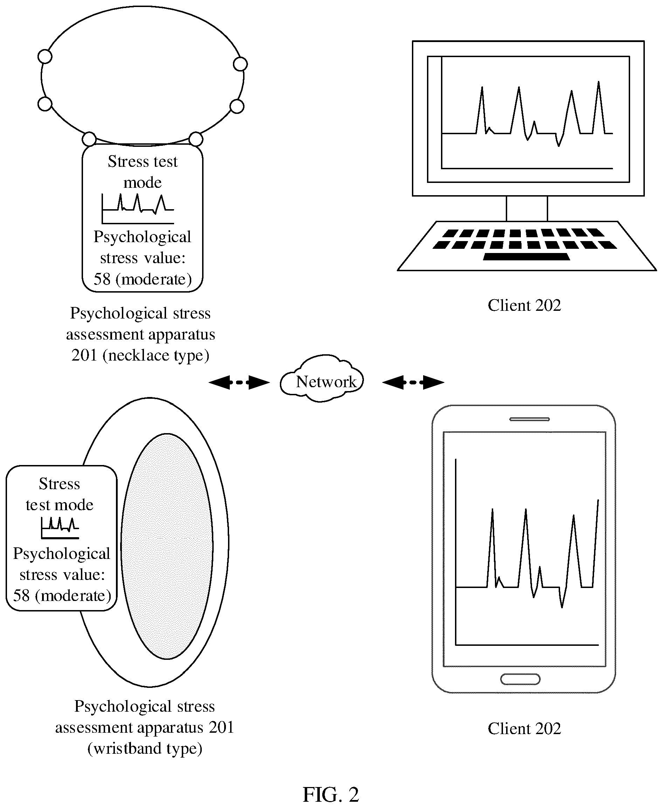

FIG. 2 is a schematic architectural diagram of a psychological stress assessment system according to an embodiment of this application. In an application scenario of psychological stress assessment, the system may include a psychological stress assessment apparatus 201 and a client 202. The psychological stress assessment apparatus 201 may communicate with the client 202 in a wired or wireless manner or in another communication manner. In this system, the psychological stress assessment apparatus 201 is mainly responsible for a psychological stress assessment function, and is mainly configured to determine a psychological status of a user. Refer to FIG. 2.

The psychological stress assessment apparatus 201 may be a terminal device that has some computing functions, that can be connected to an intelligent terminal or various terminal devices, and that exists in a form of a portable wearable accessory, for example, a wearable device (Wearable Device) that is of a watch type (including products such as a watch and a wristband) and that is supported by a wrist, a wearable device that is of a shoes type (including a shoe, a sock, or another future product worn on a leg) and that is supported by a foot, a wearable device that is of a glass type (including glasses, a helmet, and a headband) and that is supported by a head, or another wearable device that exists in a form of a headset, a necklace, smart clothing, a school bag, a crutch, an accessory, or the like; or may be a terminal device that has some computing functions and that can be connected to various devices that test physiological parameters such as a heartbeat at any time, for example, a mobile phone, a cordless telephone set, a tablet device, a handheld device having a wireless communication function, a computing device, a vehicle-mounted communications module, a smart meter, or another processing device connected to a wireless modem. Alternatively, the psychological stress assessment apparatus 201 may be a function module or the like installed in or running on any one of the foregoing devices. When the psychological stress assessment apparatus 202 is a wearable smart band, the psychological stress assessment apparatus 201 may determine a first psychological stress difference of a target user, where the first psychological stress difference is an absolute value of a difference between a first psychological stress and a second psychological stress, the first psychological stress is a psychological stress value of the target user in a first activity, the second psychological stress is a psychological stress value of the target user in a second activity, and the first activity and the second activity are activities of different types; determine a second psychological stress difference of a target crowd, where the target crowd is a crowd to which the target user belongs, the second psychological stress difference is an absolute value of a difference between a third psychological stress and a fourth psychological stress, the third psychological stress is an average psychological stress value of the target crowd in the first activity, and the fourth psychological stress is an average psychological stress value of the target crowd in the second activity; and determine a psychological stress status of the target user based on the first psychological stress difference and the second psychological stress difference.

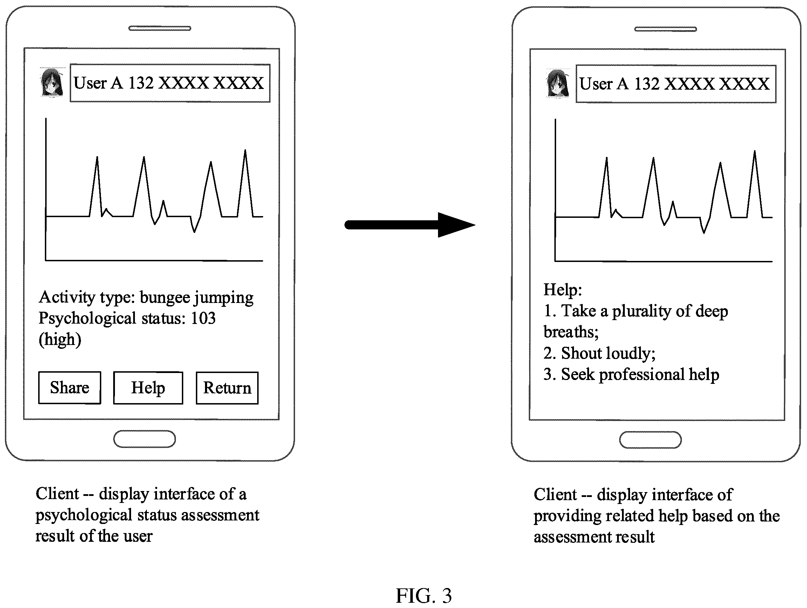

The client 202 may be a device at a network outermost periphery in a computer network, for example, a communications terminal, a mobile device, a user terminal, a mobile terminal, a wireless communications device, a portable terminal, a user agent, a user apparatus, a serving device, or user equipment (User Equipment, UE), and is mainly configured to enter data, output or display a processing result, and the like; or may be a software client, an application program, or the like installed in or running on any one of the foregoing devices. For example, the terminal may be a mobile phone, a cordless telephone set, a smartwatch, a wearable device, a tablet device, a handheld device having a wireless communication function, a computing device, a vehicle-mounted communications module, a smart meter, or another processing device connected to a wireless modem. FIG. 3 is a schematic interface diagram of an example client 202 according to an embodiment of this application, including client -- display interface of a psychological status assessment result of a user and client -- display interface of providing related help based on the assessment result. When the client 202 is a smartphone, the client 202 may perform related psychological stress health analysis based on a psychological stress state that is of a user and that is sent by the psychological stress assessment apparatus 202, or further share the psychological stress health analysis of the user with a related platform by using a specific means, so that a related person obtains a large quantity of psychological stress health analyses of users from the platform to perform related analysis and research. For example, the client may determine a psychological health degree of the user based on a psychological stress status in a specific period, to enable the user to know whether the user is at a normal level in a crowd to which the user belongs; and further recommend, in time, that the user should adjust psychological health of the user; or the client may directly recommend a related psychological health service based on psychological stress health of the user, to protect psychological and physiological health of the user.

It may be understood that the system architecture in FIG. 2 is merely an example implementation in the embodiments of the present invention. The system in the embodiments of the present invention includes but is not limited to the foregoing system architecture.

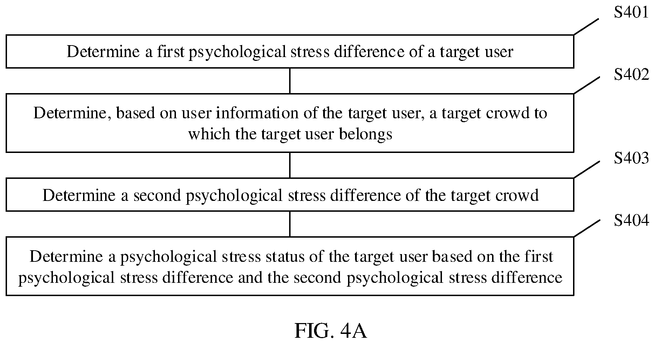

FIG. 4A is a schematic flowchart of a psychological stress assessment method according to an embodiment of this application. The method may be applied to the foregoing system in FIG. 1. The following describes the method from a single side of the psychological stress assessment apparatus 201 with reference to FIG. 4A by using an example in which an execution body is the psychological stress assessment apparatus 201. The method may include the following step S401 to step S404.

Step S401: Determine a first psychological stress difference of a target user.

Specifically, the psychological stress assessment apparatus may determine the first psychological stress difference of the target user based on a first psychological stress and a second psychological stress. The first psychological stress difference is an absolute value of a difference between the first psychological stress and the second psychological stress, the first psychological stress is a psychological stress value of the target user in a first activity, the second psychological stress is a psychological stress value of the target user in a second activity, the first activity and the second activity are activities of different types, and the activity type may include one or more of an aerobic exercise, an anaerobic exercise, a static exercise, or a dynamic exercise, for example, walking, swimming, jogging, fast running, sports competition, work, study, examination, sleep, or yoga. Considering an individual difference of a user, that is, on a basis that the user feels different psychological stresses in different activities, in this embodiment of this application, the psychological stress difference of the target user in the two activities of different types is pertinently determined. For example, the psychological stress assessment apparatus may separately determine a running psychological stress of the user A during running and a sleeping psychological stress of the user A during sleeping, and further determine a psychological stress difference between the running and the sleeping based on an absolute value of a difference between the running psychological stress and the sleeping psychological stress.

In a possible implementation, a specific determining manner of the first psychological stress is as follows: The first psychological stress is a psychological stress value determined based on a heartbeat interval sequence $T_1$ in a first time period, the first time period is a time period in which the target user performs the first activity, and the heartbeat interval sequence is a sequence including a time interval between each two heartbeats in a unit time. The second psychological stress is a psychological stress value determined based on a heartbeat interval sequence $T_2$ in a second time period, and the second time period is a time period in which the target user performs the second activity. For example, first, a plurality of heartbeat interval sequences of the user in a time period in which an activity is performed are obtained, and then a psychological status of the user is further assessed based on a fluctuation status of the plurality of heartbeat interval sequences. It may be understood that when determining the first psychological stress of the user in the first activity, the psychological stress assessment apparatus may determine the psychological stress value based on the heartbeat interval sequence $T_1$ of the user in the time period of the activity. For example, when determining the first psychological stress of the user, that is, determining the running psychological stress of the tested user A during running, the psychological stress assessment apparatus may first preset a running time of the tested user A in each five minutes after the tested user A starts running and test a heartbeat interval sequence of one minute; then determine a plurality of psychological stress values of the tested user A during running based on the plurality of heartbeat interval sequences, where the psychological stress value in this case is a psychological stress value that is of the tested user A and that is tested in one minute corresponding to a heartbeat interval sequence; and then determine the running psychological stress of the tested user A during running based on the plurality of psychological stress values after determining the plurality of psychological stress values. For example, the running psychological stress value may be determined based on the plurality of psychological stress values by using an averaging method, or it may be determined, based on a difference in running duration of the tested user A and the plurality of psychological stress values, that the running psychological stress falls within a specific range. It may be understood that, when the first psychological stress of the user in the first activity is determined, a plurality of first psychological stresses may be determined, and then an average first psychological stress in the first activity may be further determined according to, for example, a weighted averaging method. In a daily scenario, a psychological stress assessment apparatus, such as a wristband, having a heart rate collector, another heartbeat sensor, or the like may be used to assess and track a value level of a body parameter of the user. Alternatively, an apparatus, such as a wristband, having a heart rate collector, another heartbeat sensor, or the like may be used to measure and track a value level of a body parameter of the user, and then send the value level of the body parameter to a psychological stress assessment apparatus, and the psychological stress assessment apparatus further performs psychological stress assessment based on the parameter.

In a possible implementation, a specific calculation formula of the first psychological stress is as follows: $Y=\Delta d+\alpha * f(X)$, where Y is the first psychological stress, $\Delta d$ is a preset stress calibration value, $\alpha$ is a target stress gain factor, the target stress gain factor is used to represent a psychological stress change degree of the target user relative to a target crowd, f(X) is a function related to a physiological parameter X, X is determined based on M heartbeat interval sequences $T_1$ in the first time period, X represents heartbeat interval sequence variability of the target user in the first time period, and M is a positive integer. The heartbeat interval sequence is a sequence including interval duration between each two heartbeats in a preset time. Therefore, there may be the M heartbeat interval sequences $T_1$ in the first time period, and the M heartbeat interval sequences $T_1$ may be selected from consecutive preset times, or may be selected from time periods with a specific time interval. Optionally, a calculation formula of the second psychological stress is the same as the foregoing calculation formula, and a physiological parameter X corresponding to the second psychological stress is determined based on M heartbeat interval sequences $T_2$ in the foregoing second time period. It is not difficult to understand that, when determining the first psychological stress by using the formula, the psychological stress assessment apparatus may first determine, based on a plurality of heartbeat interval sequences, the physiological parameter X used to represent the heartbeat interval sequence variability, where the physiological parameter X may be a change status of a period difference between successive heartbeats, for example, the physiological parameter X may be represented as heart rate variability; then determine, based on the mapping relationship f(X) between the physiological parameter X and a psychological stress, the psychological stress corresponding to the physiological parameter; and finally determine the first psychological stress according to $Y=\Delta d+\alpha * f(X)$. Generally, the heart rate variability is mainly analyzed in terms of time domain, frequency, and nonlinearity. In terms of time domain, the heart rate variability is a standard deviation of all sinus beat RR intervals (NN intervals for short) SDNN. The SDNN is in a unit of ms, and has a normal reference value of $141 \pm 39$ and another standard value of $141.7 \pm 29.2$. In terms of frequency, the heart rate variability is a standard deviation of average RR intervals SDANN. The SDANN is in a unit of ms, and has a normal reference value of $130.9 \pm 28.3$. In terms of nonlinearity, the heart rate variability is a root mean square of adjacent RR interval differences RMSSD. The RMSSD has a normal reference value of $39.0 \pm 15.0$, and a proportion of a quantity of pairs of adjacent NN intervals with a difference >50 ms in a total quantity of sinus beats PNN50 has a normal reference value of $16.7 \pm 12.3$. For example, after the heart rate variability HRV is determined based on the plurality of heartbeat interval sequences in the first time period, heart rate variability determined for the user A is analyzed, and finally SDNN=141 ms, SDANN=130.9 ms, RMSSD=39.0, and/or PNN50=16.7 are obtained. Further, it is determined, through calculation based on a mapping relationship f(X), that a psychological stress value of the user A is 50 (it is assumed that the user A is normal when the psychological stress value is 50). In this case, the obtained psychological stress value is a psychological stress value of the user A in an activity type. Further, the psychological stress value is corrected based on a target stress gain factor of the user with reference to a status of the user. Finally, the first psychological stress is determined based on a corrected psychological stress value. Therefore, heartbeat interval sequences in different activities are used to determine psychological stresses corresponding to the activities, and the psychological stresses are used to represent a psychological stress change degree, so that the psychological status of the user can be accurately assessed, thereby improving accuracy of psychological stress assessment for the user.

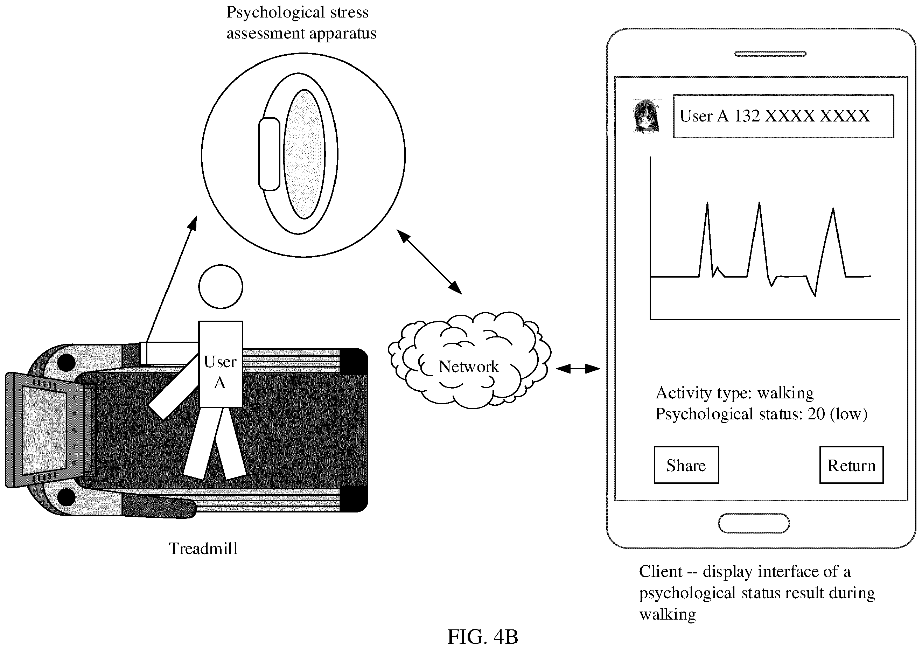

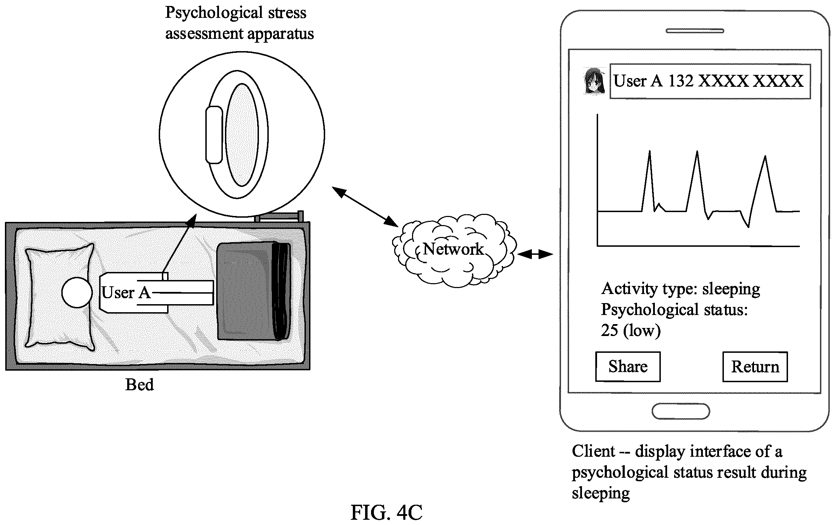

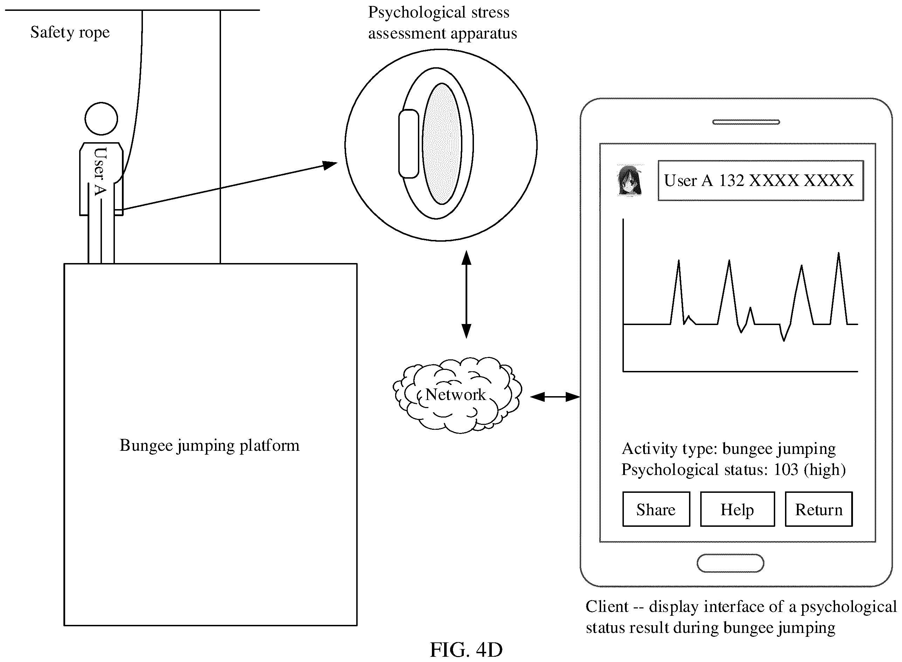

In a possible implementation, the target stress gain factor is determined based on $C_G^2$ stress gain factors, $C_G^2$ represents a combination quantity of any two of G activities of different types, the stress gain factor is a ratio of a third psychological stress difference to a fourth psychological stress difference, the third psychological stress difference is an absolute value of a difference between a fifth psychological stress and a sixth psychological stress, the fifth psychological stress and the sixth psychological stress are psychological stress values respectively corresponding to the target user in any two of the G activities, the fourth psychological stress difference is an absolute value of a difference between a seventh psychological stress and an eighth psychological stress, the seventh psychological stress and the eighth psychological stress are average psychological stress values respectively corresponding to the target crowd in the any two activities, and the G activities of different types include the first activity and the second activity, and G is a positive integer greater than or equal to 2. For example, a first stress gain factor is determined, where the first stress gain factor is used to represent a psychological stress change degree of the target user during the first activity and the second activity, and the first stress gain factor is a ratio of the first stress difference to the second stress difference; and a second stress gain factor is determined, where the second stress gain factor is used to represent a psychological stress change of the target user during a third activity and a fourth activity performed by the target user, the first activity, the second activity, the third activity, and the fourth activity are included in the G activities of different types, that is, if an activity type of the third activity is the same as that of the first activity, an activity type of the fourth activity is different from that of the second activity; or if an activity type of the third activity is the same as that of the second activity, an activity type of the fourth activity is different from that of the first activity; and then the target stress gain factor of the target user is determined based on the first stress gain factor and the second stress gain factor. It is not difficult to understand that the target stress gain factor may be determined based on a plurality of stress gain factors by using a preset rule (for example, a weighted averaging method), and the stress gain factor may be determined by randomly selecting two activities from the G activities of different types to calculate a psychological stress difference of the user, then determining a psychological stress difference of the crowd to which the user belongs in the two activities, and finally determining a ratio of the psychological stress difference of the user to the psychological stress difference of the crowd. For example, FIG. 4B is a schematic diagram of an application scenario of a user A during walking according to an embodiment of this application, FIG. 4C is a schematic diagram of an application scenario of the user A during sleeping according to an embodiment of this application, and FIG. 4D is a schematic diagram of an application scenario of the user A during bungee jumping according to an embodiment of this application. When there are three activities of different types: walking, sleeping, and bungee jumping, first, it is determined that a psychological stress difference of the user A during walking and sleeping is 5 and a psychological stress difference of a crowd to which the user belongs during walking and sleeping is 4.8, and therefore a stress gain factor of the user A during walking and sleeping is 5/4.8. Next, it is determined that a psychological stress difference of the user A during walking and bungee jumping is 83 and a psychological stress difference of the crowd to which the user belongs during walking and bungee jumping is 80.8, and therefore a stress gain factor of the user A during walking and bungee jumping is 83/80.8. Then, it is determined that a psychological stress difference of the user A during sleeping and bungee jumping is 78 and a psychological stress difference of the crowd to which the user A belongs during sleeping and bungee jumping is 76, and therefore a stress gain factor of the user A during sleeping and bungee jumping is 78/76. Finally, it is determined, based on the three stress gain factors, that a target stress gain factor is [(5/4.8)+(83/80.8)+(78/76)]/3=1.0317. The target stress gain factor is used to represent a psychological stress change degree of the target user relative to the target crowd. It may be understood that, with an increase in a quantity of types of activities that the user participates in and an increase in duration of an activity that the user participates in, a degree of correlation between the target stress gain factor and the user also increases, so that accuracy of a psychological stress value finally determined by using the target stress gain factor increases, and psychological stress assessment for the user is more accurate. It should be noted that, during initial assessment, because the psychological stress assessment apparatus has not obtained sufficient user data (for example, a heartbeat interval sequence during an activity that the user participates in), the psychological stress assessment apparatus may set the target stress gain factor to an initial value (for example, the target stress gain factor = 1) based on related research data, and subsequently update, based on obtained user data of the target user, the target stress gain factor corresponding to the target user.

Step S402: Determine, based on user information of the target user, the target crowd to which the target user belongs.

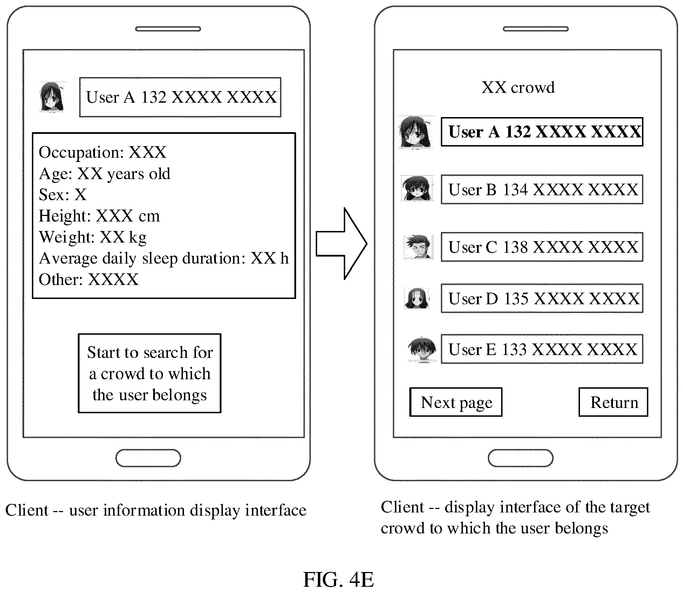

Specifically, the psychological stress assessment apparatus determines, based on the user information of the target user, the target crowd to which the target user belongs. The user information includes one or more of an occupation, an age, a sex, a height, a weight, or average daily sleep duration, or may include a body health status of the target user, or the like. FIG. 4E is a schematic diagram of an interface for determining a target crowd according to an embodiment of this application, including client -- user information display interface and client -- display interface of a target crowd to which a user belongs. Optionally, if the psychological stress assessment apparatus is a terminal device that exists in a form of a portable wearable accessory, the psychological stress assessment apparatus may be connected to a client (through, for example, Bluetooth or WiFi), so that a user of the psychological stress assessment apparatus can enter the user information of the target user to the client to determine the target crowd to which the target user belongs. It may be understood that the target user may belong to another target crowd when belonging to a target crowd, in other words, one target user may simultaneously belong to a plurality of target crowds. For example, a 65-year-old male swimmer enthusiast B has a height-to-weight ratio of 28 (for example, the height-to-weight ratio is weight (unit: Kg) divided by a square of height (unit: m), and a normal male height-to-weight ratio is 20 to 25) and has average daily sleep duration of 7 hours. Therefore, the user B may belong to an exercise crowd that regularly exercises, may also belong to an older overweight crowd, and may further belong to an older overweight crowd that regularly exercises. In addition, due to a similarity of a user crowd, a psychological stress of the target user is more similar to that of a user of a same type than a user of a different type. Therefore, when the user information is more detailed, classification of the target crowd is more accurate, and the target crowd has a higher similarity degree. Further, when the target crowd has a higher similarity degree, the target crowd also has greater reference value for the user. Therefore, in this embodiment of this application, crowd classification is performed according to a specific rule by using the user information of the user, so that the crowd to which the user belongs has a higher similarity degree when the user information is more detailed. Further, after the target crowd is determined by using the classification manner, an obtained average psychological stress of the target crowd is more accurate, and further a psychological stress that is of the user and that is determined based on the average psychological stress is also more accurate. Optionally, the user information of the target user may be updated in real time or periodically, so that the crowd to which the target user belongs may be accurately obtained through classification. For example, as the age increases and more events are experienced, psychological tolerance of the user becomes stronger, and the crowd to which the user belongs also changes.

Step S403: Determine the second psychological stress difference of the target crowd.

Specifically, the psychological stress assessment apparatus determines the second psychological stress difference of the target crowd in two activities. The target crowd is a crowd to which the target user belongs, the second psychological stress difference is an absolute value of a difference between a third psychological stress and a fourth psychological stress, the third psychological stress is an average psychological stress value of the target crowd in the first activity, the fourth psychological stress is an average psychological stress value of the target crowd in the second activity, and the two activities are the activities for determining the first psychological stress difference of the target user in step S401. Members in the crowd to which the user belongs often feel similar psychological stresses in a same activity. Therefore, then the psychological stress difference of the crowd to which the target user belongs in the two activities is determined, and finally a psychological status of the target user is accurately determined. This reduces inaccuracy of an individual difference, and improves psychological stress assessment accuracy.

In a possible implementation, the target crowd may include N users; the third psychological stress is an average psychological stress determined based on psychological stress values of the N users in a third time period, the third time period is a time period in which the N users perform the first activity, and N is a positive integer; and the fourth psychological stress is an average psychological stress value determined based on psychological stress values of the N users in a fourth time period, and the fourth time period is a time period in which the N users perform the second activity. Considering an individual difference of a user, users with a very high similarity degree in the crowd may have different psychological stresses in activities of a same activity type. Therefore, the psychological stress assessment apparatus may determine an average psychological stress of the crowd based on psychological stress values of a plurality of users in the crowd, and then determine the second psychological stress difference of the target crowd. For example, the target crowd includes other three users in addition to the target user A, and the three users are a user B, a user C, and a user D. Assuming that a third psychological stress of the user B is 44 and a fourth psychological stress of the user B is 55, a third psychological stress of the user C is 48 and a fourth psychological stress of the user C is 59, and a third psychological stress of the user C is 45 and a fourth psychological stress of the user C is 54, the second psychological stress difference of the target crowd may be $|(44 + 48 + 45) \div 3 - (55 + 59 + 54) \div 3| = 10.333$. It may be understood that the average psychological stress may be obtained by using not only a simple averaging method but also another related algorithm, provided that the average psychological stress can reasonably represent a psychological stress of the target crowd in an activity. For example, a stress range of a psychological stress of the target crowd in an activity may be determined through investigation, and then a mode or an average of stress values in the stress range is selected as an average psychological stress of the target crowd in the activity based on a quantity of members in the target crowd. Then, a psychological stress of the target user is determined by using the average psychological stress, so that psychological stress assessment for the user is more accurate.

Step S404: Determine a psychological stress status of the target user based on the first psychological stress difference and the second psychological stress difference.

Specifically, the psychological stress assessment apparatus determines the psychological stress status of the target user based on the first psychological stress difference and the second psychological stress difference. Optionally, the psychological stress status of the target user may include a psychological stress value of the target user. Alternatively, the psychological stress assessment apparatus determines a psychological health report of the target user based on a psychological stress value of the target user in a specific period, to help the user know the psychological stress status of the user in time to maintain psychological health of the target user. For example, a related psychological health service is customized based on a requirement of the user, or the psychological stress status of the user is shared with a related platform by using a specific means, so that a related person can analyze and study a psychological status of a related crowd after obtaining a large quantity of psychological stress statuses of users from the platform. In addition, the first psychological stress difference is a psychological stress difference of the target user in two activities, and the second psychological stress difference is a psychological stress difference of the target crowd in the two activities. Therefore, considering an individual difference of a user and a similarity of a user crowd, that is, on a basis that the user feels different psychological stresses in different activities, but members in the crowd to which the user belongs often feel similar psychological stresses in a same activity, when determining the first psychological stress difference of the target user, the psychological stress assessment apparatus may determine the first psychological stress difference of the target user based on the target stress gain factor that is used to represent the psychological stress change degree of the target user relative to the target crowd. With an increase in a quantity of types of activities that the user participates in and an increase in duration of an activity that the user participates in, a degree of correlation between the target stress gain factor and the user also increases, so that accuracy of the psychological stress value finally determined by using the target stress gain factor increases, and psychological stress assessment for the user is more accurate, thereby reducing inaccuracy of an individual difference. Optionally, the determining a psychological stress status of the target user based on the first psychological stress difference and the second psychological stress difference includes: determining a psychological stress of the target user based on the first psychological stress difference and the second psychological stress difference, where the psychological stress is determined based on a trained/updated target stress gain factor, and the trained/updated target stress gain factor is a stress gain factor whose degree of correlation with the user is increased after the user participates in activities of a plurality of activity types and duration in which the user participates in the activities reach a specific value.

In conclusion, in this application, in the psychological stress assessment method, first, the first psychological stress difference of the target user in the first activity and the second activity may be determined, then the second psychological stress difference of the target crowd in the same two activities may be determined, and finally the psychological stress status of the target user may be determined based on the first psychological stress difference and the second psychological stress difference. In this embodiment of this application, considering an individual difference of a user and a similarity of a user crowd, that is, on a basis that the user feels different psychological stresses in different activities, but members in the crowd to which the user belongs often feel similar psychological stresses in a same activity, first, the psychological stress difference of the target user in the two activities of different types is pertinently determined, then the psychological stress difference of the crowd to which the target user belongs in the two activities is determined, and finally the psychological status of the target user is accurately determined. This reduces inaccuracy of an individual difference, and also provides a more effective psychological stress assessment method to improve psychological stress assessment accuracy.

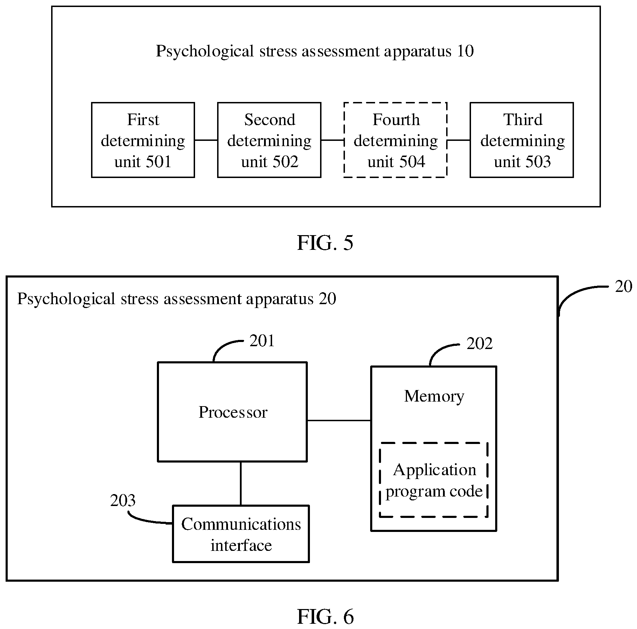

The foregoing describes the method in the embodiments of this application in detail, and the following provides a related psychological stress assessment apparatus in the embodiments of this application. The psychological stress assessment apparatus may be a terminal device that has some computing functions, that can be connected to an intelligent terminal or various terminal devices, and exists in a form of a portable accessory, or may be a terminal device that has some computing functions and that can be connected to various devices that test physiological parameters such as a heartbeat at any time. FIG. 5 is a schematic diagram of a psychological stress assessment apparatus according to an embodiment of this application. A psychological stress assessment apparatus 10 includes a first determining unit 501, a second determining unit 502, and a third determining unit 503, and may further include a fourth determining unit 504.

The first determining unit 501 is configured to determine a first psychological stress difference of a target user, where the first psychological stress difference is an absolute value of a difference between a first psychological stress and a second psychological stress, the first psychological stress is a psychological stress value of the target user in a first activity, the second psychological stress is a psychological stress value of the target user in a second activity, and the first activity and the second activity are activities of different types.

The second determining unit 502 is configured to determine a second psychological stress difference of a target crowd, where the target crowd is a crowd to which the target user belongs, the second psychological stress difference is an absolute value of a difference between a third psychological stress and a fourth psychological stress, the third psychological stress is an average psychological stress value of the target crowd in the first activity, and the fourth psychological stress is an average psychological stress value of the target crowd in the second activity.

The third determining unit 503 is configured to determine a psychological stress status of the target user based on the first psychological stress difference and the second psychological stress difference.

In a possible implementation, the first psychological stress is a psychological stress value determined based on a heartbeat interval sequence $T_1$ in a first time period, and the first time period is a time period in which the target user performs the first activity; and the second psychological stress is a psychological stress value determined based on a heartbeat interval sequence $T_2$ in a second time period, and the second time period is a time period in which the target user performs the second activity. This embodiment of this application provides a psychological assessment apparatus that obtains a heartbeat interval sequence of a target user in a time period in which an activity is performed, and then further determines a psychological stress value of the target user based on the heartbeat interval sequence. The apparatus can accurately assess a psychological status of the target user, to reduce an impact on a final assessment result due to a subjective factor of the target user, thereby improving psychological stress assessment accuracy.

In a possible implementation, a calculation formula of the first psychological stress is as follows: $Y=\Delta d+\alpha*f(X)$, where Y is the first psychological stress, $\Delta d$ is a preset stress calibration value, $\alpha$ is a target stress gain factor, the target stress gain factor is used to represent a psychological stress change degree of the target user relative to the target crowd, f(X) is a function related to a physiological parameter X, X is determined based on M heartbeat interval sequences $T_1$ in the first time period, X represents heartbeat interval sequence variability of the target user in the first time period, and M is a positive integer.

In a possible implementation, the target stress gain factor is determined based on $C_G^2$ stress gain factors, $C_G^2$ represents a combination quantity of any two of G activities of different types, the stress gain factor is a ratio of a third psychological stress difference to a fourth psychological stress difference, the third psychological stress difference is an absolute value of a difference between a fifth psychological stress and a sixth psychological stress, the fifth psychological stress and the sixth psychological stress are psychological stress values respectively corresponding to the target user in any two of the G activities, the fourth psychological stress difference is an absolute value of a difference between a seventh psychological stress and an eighth psychological stress, the seventh psychological stress and the eighth psychological stress are average psychological stress values respectively corresponding to the target crowd in the any two activities, and the G activities of different types include the first activity and the second activity, and G is a positive integer greater than or equal to 2.

In a possible implementation, the apparatus further includes the fourth determining unit 504, configured to: before the second psychological stress difference of the target crowd is determined, determine, based on user information of the target user, the target crowd to which the target user belongs, where the user information includes one or more of an occupation, an age, a sex, a height, a weight, or average daily sleep duration. In a possible implementation, the target crowd includes N users; the third psychological stress is an average psychological stress determined based on psychological stress values of the N users in a third time period, the third time period is a time period in which the N users perform the first activity, and N is a positive integer; and the fourth psychological stress is an average psychological stress value determined based on psychological stress values of the N users in a fourth time period, and the fourth time period is a time period in which the N users perform the second activity.

It should be noted that, for functions of the function units in the psychological stress assessment apparatus 10 described in this embodiment of this application, refer to the related descriptions of step S401 to step S404 in the foregoing method embodiment in FIG. 4A. Details are not described herein again.

FIG. 6 is a schematic structural diagram of another psychological stress assessment apparatus according to an embodiment of this application. An apparatus 20 includes at least one processor 201, at least one memory 202, and at least one communications interface 203. In addition, the device may further include general-purpose components such as an antenna, and details are not described herein.

The processor 201 may be a general-purpose central processing unit (CPU), a microprocessor, an application-specific integrated circuit (application-specific integrated circuit, ASIC), or one or more integrated circuits for controlling program execution of the foregoing solutions.

The communications interface 203 is configured to communicate with another device or a communications network, such as the Ethernet, a radio access network (RAN), a core network, or a wireless local area network (Wireless Local Area Networks, WLAN).

The memory 202 may be but is not limited to a read-only memory (read-only memory, ROM) or another type of static storage device that can store static information and instructions, or a random access memory (random access memory, RAM) or another type of dynamic storage device that can store information and instructions; or may be an electrically erasable programmable read-only memory (Electrically Erasable Programmable Read-Only Memory, EEPROM), a compact disc read-only memory (Compact Disc Read-Only Memory, CD-ROM) or another compact disc storage, an optical disc storage (including a compact optical disc, a laser disc, an optical disc, a digital versatile disc, a Blu-ray disc, or the like), a magnetic disk storage medium or another magnetic storage device, or any other media that can be configured to carry or store expected program code in a form of an instruction or a data structure and that can be accessed by a computer. The memory may exist independently and is connected to the processor by using a bus. Alternatively, the memory may be integrated into the processor.

The memory 202 is configured to store application program code for executing the foregoing solutions, and the processor 201 controls the execution. The processor 201 is configured to execute the application program code stored in the memory 202.

The code stored in the memory 202 may be used to execute the foregoing psychological stress assessment method provided in FIG. 4A, for example, determine a first psychological stress difference of a target user, where the first psychological stress difference is an absolute value of a difference between a first psychological stress and a second psychological stress, the first psychological stress is a psychological stress value of the target user in a first activity, the second psychological stress is a psychological stress value of the target user in a second activity, and the first activity and the second activity are activities of different types; determine a second psychological stress difference of a target crowd, where the target crowd is a crowd to which the target user belongs, the second psychological stress difference is an absolute value of a difference between a third psychological stress and a fourth psychological stress, the third psychological stress is an average psychological stress value of the target crowd in the first activity, and the fourth psychological stress is an average psychological stress value of the target crowd in the second activity; and determine a psychological stress status of the target user based on the first psychological stress difference and the second psychological stress difference.

It should be noted that, for functions of the function units in the psychological stress assessment apparatus 20 described in this embodiment of this application, refer to the related descriptions of step S401 to step S404 in the foregoing method embodiment in FIG. 4A. Details are not described herein again.

In the foregoing embodiments, the descriptions of the embodiments have respective focuses. For a part that is not described in detail in an embodiment, refer to related descriptions in other embodiments.

It should be noted that, for brief description, the foregoing method embodiments are represented as a combination of a series of actions. However, persons skilled in the art should appreciate that this application is not limited to the described order of the actions, because according to this application, some steps may be performed in other orders or simultaneously. In addition, persons skilled in the art also should appreciate that the embodiments described in this specification are all example embodiments, and the involved actions and modules are not necessarily required by this application.

In the several embodiments provided in this application, it should be understood that the disclosed apparatus may be implemented in other manners. For example, the described apparatus embodiment is merely an example. For example, the unit division is merely logical function division and may be other division in actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in electronic or other forms.

The foregoing units described as separate parts may or may not be physically separate, and parts displayed as units may or may not be physical units, that is, may be located in one position, or may be distributed in a plurality of network units. Some or all of the units may be selected based on actual requirements to achieve the objectives of the solutions of the embodiments.

In addition, the function units in the embodiments of this application may be integrated into one processing unit, or each of the units may exist alone physically, or two or more units are integrated into one unit. The integrated unit may be implemented in a form of hardware, or may be implemented in a form of a software function unit.

When the foregoing integrated unit is implemented in the form of a software function unit and sold or used as an independent product, the integrated unit may be stored in a computer-readable storage medium. Based on such an understanding, the technical solutions of this application essentially, or the part contributing to the prior art, or all or some of the technical solutions may be implemented in a form of a software product. The computer software product is stored in a storage medium and includes several instructions for instructing a computer device (which may be a personal computer, a serving end, a network device, or the like) to perform all or some of the steps of the methods described in the embodiments of this application. The foregoing storage medium may include any medium that can store program code, such as a USB flash drive, a removable hard disk, a magnetic disk, an optical disc, a read-only memory (Read-Only Memory, ROM for short), or a random access memory (Random Access Memory, RAM for short).

The foregoing embodiments are merely intended for describing the technical solutions of this application, but not for limiting this application. Although this application is described in detail with reference to the foregoing embodiments, persons of ordinary skill in the art should understand that they may still make modifications to the technical solutions described in the foregoing embodiments or make equivalent replacements to some technical features thereof, without departing from the spirit and scope of the technical solutions of the embodiments of this application.

**Claims**

1. A psychological stress assessment method, comprising:

   determining a first psychological stress difference of a target user, wherein the first psychological stress difference is an absolute value of a difference between a first psychological stress and a second psychological stress, the first psychological stress is a psychological stress value of the target user in a first activity, the second psychological stress is a psychological stress value of the target user in a second activity, and the first activity and the second activity are activities of different types;
   determining a second psychological stress difference of a target crowd, wherein the target crowd is a crowd to which the target user belongs, the second psychological stress difference is an absolute value of a difference between a third psychological stress and a fourth psychological stress, the third psychological stress is an average psychological stress value of the target crowd in the first activity, and the fourth psychological stress is an average psychological stress value of the target crowd in the second activity; and
   determining a psychological stress status of the target user based on the first psychological stress difference and the second psychological stress difference.

2. The method according to claim 1, wherein the first psychological stress is a psychological stress value determined based on a heartbeat interval sequence $T_1$ in a first time period, and the first time period is a time period in which the target user performs the first activity; and
   the second psychological stress is a psychological stress value determined based on a heartbeat interval sequence $T_2$ in a second time period, and the second time period is a time period in which the target user performs the second activity.

3. The method according to claim 2, wherein a calculation formula of the first psychological stress is as follows:
   $Y=\Delta d+\alpha*f(X)$, wherein Y is the first psychological stress, $\Delta d$ is a preset stress calibration value, $\alpha$ is a target stress gain factor, the target stress gain factor is used to represent a psychological stress change degree of the target user relative to the target crowd, $f(X)$ is a function related to a physiological parameter X, X is determined based on M heartbeat interval sequences $T_1$ in the first time period, X represents heartbeat interval sequence variability of the target user in the first time period, and M is a positive integer.

4. The method according to claim 3, wherein the target stress gain factor is determined based on $C_G^2$ stress gain factors, $C_G^2$ represents a combination quantity of any two of G activities of different types, the stress gain factor is a ratio of a third psychological stress difference to a fourth psychological stress difference, the third psychological stress difference is an absolute value of a difference between a fifth psychological stress and a sixth psychological stress, the fifth psychological stress and the sixth psychological stress are psychological stress values respectively corresponding to the target user in any two of the G activities, the fourth psychological stress difference is an absolute value of a difference between a seventh psychological stress and an eighth psychological stress, the seventh psychological stress and the eighth psychological stress are average psychological stress values respectively corresponding to the target crowd in the any two activities, and the G activities of different types comprise the first activity and the second activity, and G is a positive integer greater than or equal to 2.

5. The method according to any one of claims 1 to 4, wherein before the determining a second psychological stress difference of a target crowd, the method further comprises:
   determining, based on user information of the target user, the target crowd to which the target user belongs, wherein the user information comprises one or more of an occupation, an age, a sex, a height, a weight, or average daily sleep duration.

6. The method according to any one of claims 1 to 4, wherein the target crowd comprises N users;

   the third psychological stress is an average psychological stress determined based on psychological stress values of the N users in a third time period, the third time period is a time period in which the N users perform the first activity, and N is a positive integer; and

the fourth psychological stress is an average psychological stress value determined based on psychological stress values of the N users in a fourth time period, and the fourth time period is a time period in which the N users perform the second activity.

7. A psychological stress assessment apparatus, comprising:

a first determining unit, configured to determine a first psychological stress difference of a target user, wherein the first psychological stress difference is an absolute value of a difference between a first psychological stress and a second psychological stress, the first psychological stress is a psychological stress value of the target user in a first activity, the second psychological stress is a psychological stress value of the target user in a second activity, and the first activity and the second activity are activities of different types;

a second determining unit, configured to determine a second psychological stress difference of a target crowd, wherein the target crowd is a crowd to which the target user belongs, the second psychological stress difference is an absolute value of a difference between a third psychological stress and a fourth psychological stress, the third psychological stress is an average psychological stress value of the target crowd in the first activity, and the fourth psychological stress is an average psychological stress value of the target crowd in the second activity; and

a third determining unit, configured to determine a psychological stress status of the target user based on the first psychological stress difference and the second psychological stress difference.

8. The apparatus according to claim 7, wherein the first psychological stress is a psychological stress value determined based on a heartbeat interval sequence $T_1$ in a first time period, and the first time period is a time period in which the target user performs the first activity; and

the second psychological stress is a psychological stress value determined based on a heartbeat interval sequence $T_2$ in a second time period, and the second time period is a time period in which the target user performs the second activity.

9. The apparatus according to claim 8, wherein a calculation formula of the first psychological stress is as follows:

$Y=\Delta d+\alpha*f(X)$, wherein Y is the first psychological

stress, $\Delta d$ is a preset stress calibration value, $\alpha$ is a target stress gain factor, the target stress gain factor is used to represent a psychological stress change degree of the target user relative to the target crowd, f(X) is a function related to a physiological parameter X, X is determined based on M heartbeat interval sequences $T_1$ in the first time period, X represents heartbeat interval sequence variability of the target user in the first time period, and M is a positive integer.

10. The apparatus according to claim 9, wherein the target stress gain factor is determined based on $C_G^2$ stress gain factors, $C_G^2$ represents a combination quantity of any two of G activities of different types, the stress gain factor is a ratio of a third psychological stress difference to a fourth psychological stress difference, the third psychological stress difference is an absolute value of a difference between a fifth psychological stress and a sixth psychological stress, the fifth psychological stress and the sixth psychological stress are psychological stress values respectively corresponding to the target user in any two of the G activities, the fourth psychological stress difference is an absolute value of a difference between a seventh psychological stress and an eighth psychological stress, the seventh psychological stress and the eighth psychological stress are average psychological stress values respectively corresponding to the target crowd in the any two activities, and the G activities of different types comprise the first activity and the second activity, and G is a positive integer greater than or equal to 2.

11. The apparatus according to any one of claims 7 to 10, further comprising:
a fourth determining unit, configured to: before the second psychological stress difference of the target crowd is determined, determine, based on user information of the target user, the target crowd to which the target user belongs, wherein the user information comprises one or more of an occupation, an age, a sex, a height, a weight, or average daily sleep duration.

12. The apparatus according to any one of claims 7 to 10, wherein the target crowd comprises N users;

the third psychological stress is an average psychological stress determined based on psychological stress values of the N users in a third time period, the third time period is a time period in which the N users perform the first activity, and N is a positive integer; and

the fourth psychological stress is an average psychological stress value determined based on psychological stress values of the N users in a fourth time period, and the fourth time period is a time period in which the N users perform the second activity.

13. A terminal device, comprising a processor, a memory, and a communications interface, wherein the memory is configured to store information sending program code, and the processor is configured to invoke the psychological stress assessment program code to execute the method according to any one of claims 1 to 6.

14. A chip system, wherein the chip system comprises at least one processor, memory, and interface circuit, the memory, the interface circuit, and the at least one processor are interconnected by using a line, the at least one memory stores an instruction, and the method according to any one of claims 1 to 6 is implemented when the instruction is executed by the processor.

15. A computer storage medium, wherein the computer storage medium stores a computer program, and the method according to any one of claims 1 to 6 is implemented when the computer program is executed by a processor.

16. A computer program, wherein the computer program comprises an instruction, and when the computer program is executed by a computer, the computer is enabled to execute the method according to any one of claims 1 to 6.

FIG. 1

Stress test
mode

Psychological
stress value:
58 (moderate)

Psychological stress
assessment apparatus
201 (necklace type)

Client 202

Network

Stress
test mode

Psychological
stress value:
58 (moderate)

Psychological stress
assessment apparatus 201
(wristband type)

Client 202

FIG. 2

EP 3 918 996 A1

User A 132 XXXX XXXX

Activity type: bungee jumping
Psychological status: 103
(high)

| Share | Help | Return |

Client -- display interface of a
psychological status assessment
result of the user

User A 132 XXXX XXXX

Help:
1. Take a plurality of deep
breaths;
2. Shout loudly;
3. Seek professional help

Client -- display interface of
providing related help based on the
assessment result

FIG. 3

Determine a first psychological stress difference of a target user ⟋ S401

Determine, based on user information of the target user, a target crowd to which the target user belongs ⟋ S402

Determine a second psychological stress difference of the target crowd ⟋ S403

Determine a psychological stress status of the target user based on the first psychological stress difference and the second psychological stress difference ⟋ S404

FIG. 4A

Psychological stress
assessment apparatus

User A

Network

Treadmill

User A 132 XXXX XXXX

Activity type: walking
Psychological status: 20 (low)

Share          Return

Client -- display interface of a
psychological status result during
walking

FIG. 4B

EP 3 918 996 A1

Psychological stress
assessment apparatus

Network

User A 132 XXXX XXXX

Activity type: sleeping
Psychological status:
25 (low)

Share    Return

Bed

Client -- display interface of a
psychological status result during
sleeping

FIG. 4C

Safety rope

Psychological stress assessment apparatus

User A

Bungee jumping platform

Network

User A 132 XXXX XXXX

Activity type: bungee jumping
Psychological status: 103 (high)

| Share | Help | Return |

Client -- display interface of a psychological status result during bungee jumping

FIG. 4D

EP 3 918 996 A1

User A 132 XXXX XXXX

Occupation: XXX
Age: XX years old
Sex: X
Height: XXX cm
Weight: XX kg
Average daily sleep duration: XX h
Other: XXXX

Start to search for
a crowd to which
the user belongs

XX crowd

**User A 132 XXXX XXXX**

User B 134 XXXX XXXX

User C 138 XXXX XXXX

User D 135 XXXX XXXX

User E 133 XXXX XXXX

Next page          Return

Client -- user information display interface

Client -- display interface of the target
crowd to which the user belongs

FIG. 4E

Psychological stress assessment apparatus 10

| First determining unit 501 | Second determining unit 502 | Fourth determining unit 504 | Third determining unit 503 |

**FIG. 5**

Psychological stress assessment apparatus 20

20

201

202

Processor

Memory

Application program code

203

Communications interface

**FIG. 6**

## INTERNATIONAL SEARCH REPORT

| International application No. |
|---|
| **PCT/CN2020/079153** |

| A. | CLASSIFICATION OF SUBJECT MATTER |
|---|---|

A61B 5/16(2006.01)i;  A61B 5/024(2006.01)i

According to International Patent Classification (IPC) or to both national classification and IPC

| B. | FIELDS SEARCHED |
|---|---|

Minimum documentation searched (classification system followed by classification symbols)

A61B5/-

Documentation searched other than minimum documentation to the extent that such documents are included in the fields searched

Electronic data base consulted during the international search (name of data base and, where practicable, search terms used)

CNABS, CNTXT, WPI, EPODOC, CNKI: 心理, 压力, 差, 比值, 比率, 心跳, 心率, 二 3d活动, psychological, stress, assessment, difference, heart beat?, two, activities

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 110025321 A (HUAWEI TECHNOLOGIES CO., LTD.) 19 July 2019 (2019-07-19) claims 1-16, description, paragraphs [0041]-[0097], and figures 1-6 | 1-16 |
| X | CN 108135548 A (MEDIBIO LIMITED) 08 June 2018 (2018-06-08) description, paragraphs [0001] and [0049]-[0074], and figure 4(b) | 1, 2, 5-8, 11-16 |
| A | JP 2014140398 A (LION CORP.) 07 August 2014 (2014-08-07) entire document | 1-16 |
| A | US 10213145 B1 (CERNER INNOVATION, INC.) 26 February 2019 (2019-02-26) entire document | 1-16 |
| A | US 2014005947 A1 (KOREA ELECTRONICS TECHNOLOGY INSTITUTE) 02 January 2014 (2014-01-02) entire document | 1-16 |
| A | CN 103764021 A (NANYANG TECHNOLOGICAL UNIVERSITY) 30 April 2014 (2014-04-30) entire document | 1-16 |

☐ Further documents are listed in the continuation of Box C.       ☑ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **20 May 2020** | **15 June 2020** |

| Name and mailing address of the ISA/CN | Authorized officer |
|---|---|
| **China National Intellectual Property Administration** **No. 6, Xitucheng Road, Jimenqiao Haidian District, Beijing 100088** **China** | |
| Facsimile No. **(86-10)62019451** | Telephone No. |

Form PCT/ISA/210 (second sheet) (January 2015)

**INTERNATIONAL SEARCH REPORT**
Information on patent family members

International application No.

**PCT/CN2020/079153**

| Patent document cited in search report | | | Publication date (day/month/year) | Patent family member(s) | | | Publication date (day/month/year) |
|---|---|---|---|---|---|---|---|
| CN | 110025321 | A | 19 July 2019 | None | | | |
| CN | 108135548 | A | 08 June 2018 | JP | 2018524750 | A | 30 August 2018 |
| | | | | WO | 2016201500 | A1 | 22 December 2016 |
| | | | | CA | 2988419 | A1 | 22 December 2016 |
| | | | | AU | 2016278357 | A1 | 04 January 2018 |
| | | | | US | 9861308 | B2 | 09 January 2018 |
| | | | | US | 2018184960 | A1 | 05 July 2018 |
| | | | | US | 2017156657 | A1 | 08 June 2017 |
| | | | | EP | 3307166 | A1 | 18 April 2018 |
| | | | | IL | 256151 | D0 | 28 February 2018 |
| JP | 2014140398 | A | 07 August 2014 | None | | | |
| US | 10213145 | B1 | 26 February 2019 | None | | | |
| US | 2014005947 | A1 | 02 January 2014 | US | 2015142332 | A1 | 21 May 2015 |
| CN | 103764021 | A | 30 April 2014 | US | 2014200432 | A1 | 17 July 2014 |
| | | | | EP | 2709522 | A1 | 26 March 2014 |
| | | | | PL | 2709522 | T3 | 31 March 2017 |
| | | | | CN | 103764021 | B | 25 November 2015 |
| | | | | PT | 2709522 | T | 30 November 2016 |
| | | | | MY | 167168 | A | 13 August 2018 |
| | | | | WO | 2012161657 | A1 | 29 November 2012 |
| | | | | AU | 2012259507 | B2 | 25 August 2016 |
| | | | | EP | 2709522 | B1 | 14 September 2016 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201910216114